Europäisches Patentamt

European Patent Office

Office européen des brevets

(11) Publication number: **0 186 435**
**A2**

## (12) EUROPEAN PATENT APPLICATION

(21) Application number: **85309242.7**

(22) Date of filing: **18.12.85**

(51) Int. Cl.⁴: **A 61 B 17/12**
**A 61 M 25/00**

(30) Priority: **19.12.84 JP 192687/84**

(43) Date of publication of application:
**02.07.86 Bulletin 86/27**

(84) Designated Contracting States:
**BE DE FR GB IT NL**

(71) Applicant: **Nippon Sherwood Medical Industries Limited**
**5-27-7, Sendagaya**
**Shibuya-ku Tokyo(JP)**

(72) Inventor: **Okada, Yosuke c/o Nippon Sherwood**
**Medical Industries Limited 5-27-7, Sendagaya**
**Shibuya-ku Tokyo(JP)**

(74) Representative: **Chettle, Adrian John et al,**
**c/o John Wyeth & Brother Limited Huntercombe Lane**
**South**
**Taplow Maidenhead Berkshire, SL6 0PH(GB)**

(54) Balloon tube for treating oesophageal varices.

(57) A balloon tube for treating oesophageal varices has a balloon (3) and inflation lumen (2) to close the oesophagus, and a suction lumen (1) having distal openings (5) to remove air and body fluids. Additional suction openings (5') may be provided proximal of the balloon (3) to suck in saliva and like fluids collecting in the gullet. The distal end of the tube may carry a soft outer covering tube (6) to prevent damage to the oesophagus or stomach. A stomach balloon (7) may be provided distally of the oesophagus balloon to provide positive blood checking in cases where blood loss is great.

FIG.1

EP 0 186 435 A2

## BALLOON TUBE FOR TREATING OESOPHAGEAL VARICES

The present invention relates to a balloon tube for treating oesophageal varices.

Oesophageal varices are a medical condition in which varicose veins from the lower portion of the oesophagus may rupture or otherwise leak blood into the oesophagus. Blood loss may be sufficiently great to place the patient's life in danger. One method of treatment is to inject the veins with a sclerosing fluid which causes the veins to close and thereby stop blood loss. A balloon catheter may be inflated in the affected area to press on the oesophageal wall and thereby close off the bleeding varices whilst the sclerotherapy takes effect.

Normally the oesophagus is closed and it is necessary to feed air into the oesophageal tract to open the oesophagus so that the surgeon can inspect the walls of the gullet by endoscope. Once the affected area has been identified the surgeon can inject a sclerosing fluid into the varices.

The introduction of air under pressure to inflate the oesophagus may cause some pain to the patient. Once the balloon catheter has been inflated the residual pressure in the stomach may remain. This may continue to cause the patient discomfort and in some cases the operation must be abandoned and repeated.

One object of the present invention is to provide a balloon tube for treating oesophageal varices which substantially eliminates the problems of prior art methods and provides other advantages.

According to the invention there is provided a balloon tube for treating oesophageal varices and comprising a tube having an oesophageal balloon at or near the distal end thereof and an inflation lumen for attachment to an aeration source, the tube further including a suction lumen for attachment to a suction source and having one or more suction apertures at or near the distal end of the tube.

The balloon tube according to the invention can be inflated in the affected area of the oesophagus after injection sclerotherapy and the integral suction lumen removes residual air from the stomach together with blood and other body fluids.

In a preferred embodiment one or more of the suction apertures are provided distally of the balloon and in a second preferred embodiment one or more additional suction apertures are provided proximal of the balloon. This latter arrangement ensures that saliva and other body fluids draining down the gullet will be removed and not collect on the proximal side of the balloon.

The balloon tube must have sufficient stiffness to ensure that it passes through the oesophagus into the stomach and consequently is made of a somewhat hard plastic e.g. polyvinylchloride, having a Shore hardness D of 50-60. The hard plastic tip of the tube may damage the delicate lining of the oesophagus and stomach; the tube typically has an outside diameter of 6 to 10 FR.

In another preferred embodiment of the invention the balloon tube is provided with a relatively soft covering which reduces the possibility of damage to the oesophageal and stomach wall. The soft covering is preferably a plastics outer tube and is provided around the oesophageal tube; the balloon is mounted outside thereof. An aeration passage extends through the wall of the tube and the soft covering into the balloon. In the preferred embodiment the soft covering is generally cylindrical and extends beyond the distal end of the oesophageal tube; the covering may be open ended. The suction lumen of the oesophageal tube may open into such an extended portion for collection of body fluids therefrom and additional openings may be provided in the wall of said extended portion. The soft plastics covering for the tip of the tube may be made of polyvinylchloride having a Shore hardness A of 60-80.

In a further embodiment of the invention a stomach balloon is provided distally of the oesophageal tract balloon to provide for positive blood checking. The additional balloon is especially useful where blood loss is great. The soft covering tube may extend distally of the stomach balloon.

Preferably the balloon tube has as small a diameter as possible so as not to impair the field of vision of an endoscope and so as not to interfere with injection of the varices with a sclerosing fluid.

The invention will now be described by reference to several preferred embodiments shown by way of example only in the accompanying drawings in which:-

Figure 1 is an explanatory view of a balloon tube for treating oesophageal varices in accordance with the present invention;

Figure 2 is a sectional view taken on line X-X of Figure 1;

Figure 3 is an enlarged sectional view of the portion of Figure 1 marked "Y";

Figure 4 is an explanatory view of a further embodiment;

Figure 5 is an explanatory view of yet another embodiment;

Figure 6 is a sectional view in the neighbourhood of the balloon of a tube similar to that shown in Figure 5;

Figure 7 is an explanatory view of yet another embodiment; and

Figure 8 is a sectional view taken on line Z-Z of Figure 7.

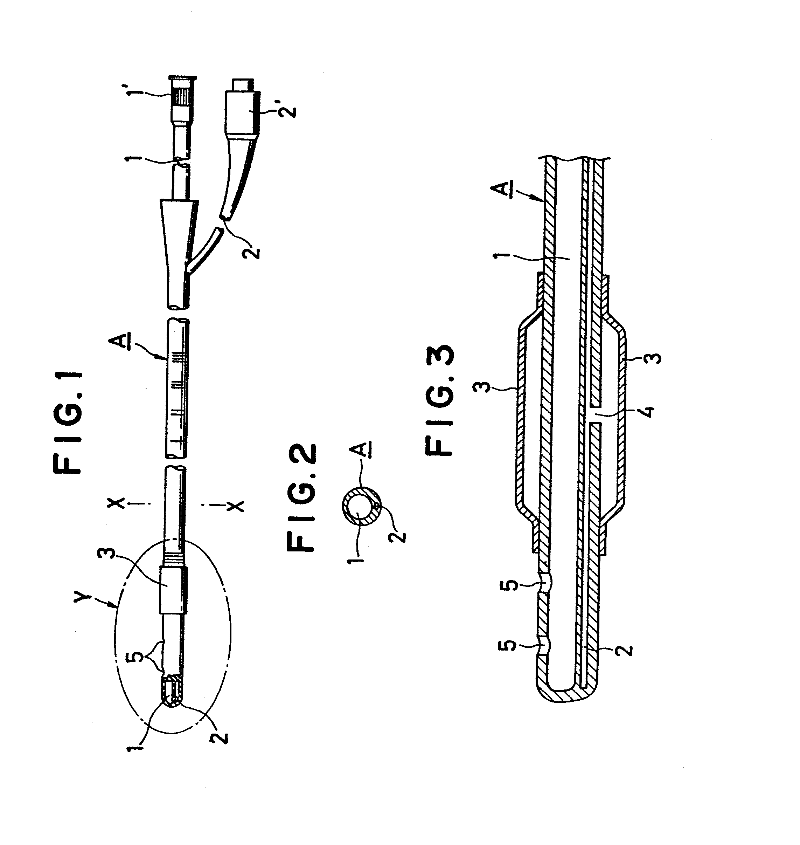

With references to Figures 1 to 3 there is shown a balloon tube for treating oesophageal varices. The tube is of small diameter, outside diameter is typically in the range 2.7 to 4.0mm, and is formed of a somewhat hard plastic having a Shore hardness D of 50-60. The tube may be principally formed of polyvinylchloride, the amount of plasticides being in the range of 30-40 PHR (parts per hundred resin).

The tube has a main lumen 1 having connection 1' to a suction source. One or more suction apertures 5 are provided at the distal end of the tube to suck in air, blood and other body fluids.

A balloon 3 is mounted near the distal end of the tube and is connected by an aperture 4 to an aeration lumen 2 which extends the full length of the tube. The lumen 2 has a connection 2' to an aeration device. Air or other fluid is introduced through the lumen 2 to inflate the balloon, for example using a syringe.

In this way residual air and body fluids can be removed through the suction lumen 1 as desired, whilst the inflated balloon presses on the oesophageal wall to close off the bleeding varices. After compression of the injection site for a suitable period the balloon is deflated and withdrawn. The surgeon may inspect the oesophagus and repeat the operation if further varices are found.

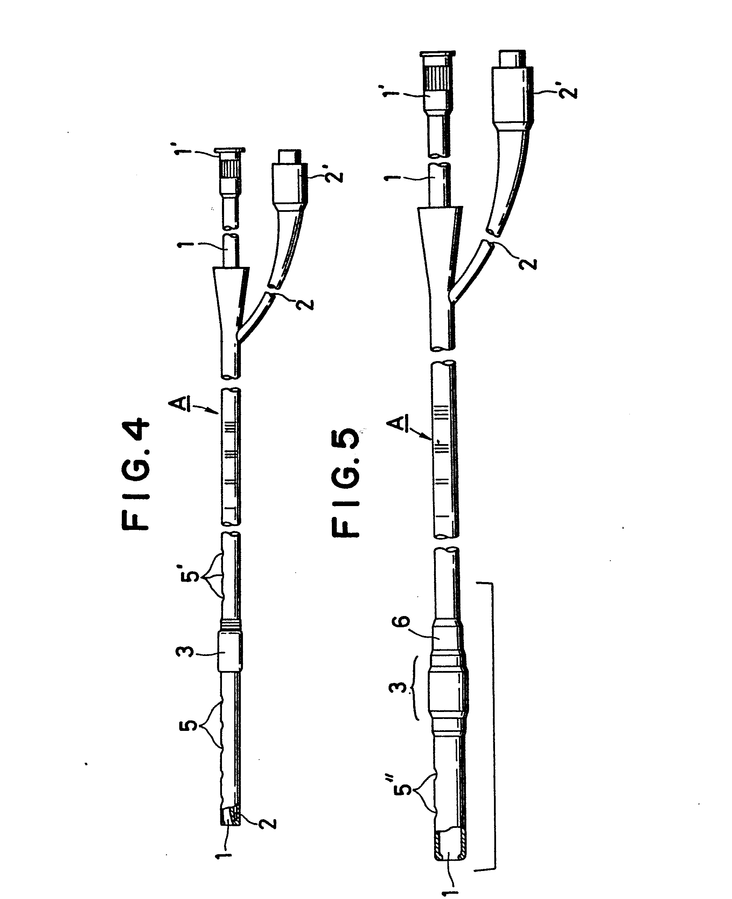

An alternative embodiment is shown in Figure 4 in which additional suction apertures 5' are provided proximally of the balloon. Such apertures 5' prevent a build up of saliva and like fluids proximal of the inflated balloon. Such fluids will otherwise collect in the oesophagus and cause discomfort to the patient.

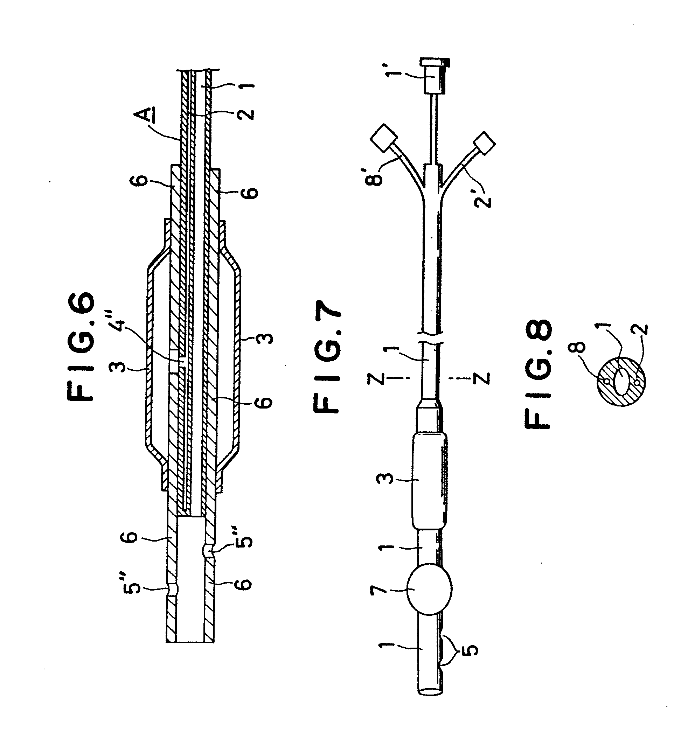

In the embodiment shown in Figures 5 and 6 the tube is provided with a relatively soft plastics covering so as to reduce the likelihood of damage to the oesophagus by the small diameter distal end of the tube.

The soft covering tube 6 is principally formed of polyvinylchloride 60-90 PHR, Shore hardness A 60-80, and extends beyond the distal end of the main tube as shown. The distal end of the suction lumen 1 is open and suction apertures 5'' are provided in the wall of the soft tube 6. Body fluids may be sucked up either through the open end of the tube 6 or through the apertures 5''. The aeration aperture 4'' extends through the soft tube as shown.

The soft covering reduces the likelihood of damage to the oesophageal wall or to the stomach wall.

In the further embodiment of Figures 7 and 8, a stomach balloon 7 is provided distally of the oesophageal balloon 3. An aeration lumen 8 connects the stomach balloon to a connector 8' for attachment to an aeration source. A soft covering tube, of the kind shown in Figures 5 and 6, may be provided distally of the stomach balloon to reduce the likelihood of damage to the stomach wall.

The stomach balloon provides a positive check where blood loss from the varicose veins in the lower portion of the oesophagus is great.

The invention thus provides means for closing the oesophagus to effectively treat oesophageal varices whilst allowing residual air and body fluids to be removed through a suction lumen.

CLAIMS

1. A balloon tube for treating oesophageal varices and comprising a plastics tube having an oesophageal balloon (3) at or near the distal end thereof and an inflation lumen (2) characterised thereby that a suction lumen (1) is provided having one or more suction apertures at or near the distal end of the tube.

2. A balloon tube according to Claim 1, characterised thereby that one or more of said suction apertures (5) are provided distally of the balloon (3).

3. A balloon tube according to Claim 2, characterised thereby that one or more suction apertures (5') are provided proximally of the balloon.

4. A balloon tube according to any preceding claim, characterised thereby that a stomach balloon (7) is mounted distally of said oesophageal balloon (3), an inflation lumen (8) being provided for said stomach balloon (7).

5. A balloon tube according to Claim 4, characterised thereby that one or more suction apertures are provided distally of the stomach balloon (7).

6. A balloon tube according to any preceding claim, characterised thereby that the distal end of said tube is covered by an outer tube (6) of relatively soft plastics material.

7. A balloon tube according to Claim 6, characterised thereby that the balloon tube is of polyvinylchloride having a Shore hardness D of 50-60 and said outer tube 6 is of polyvinylchloride having a Shore hardness A of 60-80.

8. A balloon tube according to Claim 6 or Claim 7, characterised thereby that said outer tube (6) extends distally of the distal end of said tube.

9. A balloon tube according to Claim 8, characterised thereby that said outer tube (6) has an open end.

10. A balloon tube according to Claim 8 or Claim 9, characterised thereby that suction apertures (5'') are provided in the wall of said outer tube (6).

# FIG.1

# FIG.2

# FIG.3

0186435

# FIG.4

# FIG.5

FIG.6

FIG.7

FIG.8